# EUROPEAN PATENT APPLICATION

(11) **EP 3 438 122 A1**
(43) Date of publication of application: **06.02.2019**
(21) Application number: 17184232.1
(22) Date of filing: 01.08.2017
(51) Int. Cl.: C07K 14/245, C07K 14/435, C12P 19/18, C12N 9/10, A23L 33/00

(54) **MICROORGANISM FOR PRODUCING HUMAN MILK OLIGOSACCHARIDE**

(71) Applicant: OligoScience Biotechnology GmbH, 59387 Ascheberg (DE)
(72) Inventor: The designation of the inventor has not yet been filed
(74) Representative: Hoffmann Eitle

(57) **Abstract**

Human milk oligosaccharides (HMOs) may be used e.g. as functional ingredients in infant nutrition, medical nutrition, functional foods and animal feed. There is still a need of improved means of producing HMOs. The present invention provides genetically modified microorganisms for the improved production of HMOs and HMO production methods using the same. The microorganisms of the invention may have one or more yield-enhancing modifications, including an inducible lysis system, which allows for the easy extraction of intracellular and extracellular HMOs, lactose permease mutants, which may increase intracellular lactose levels, or chaperones, which may increase intracellular availability of key enzymes for the production of HMOs.

SEQUENCE LISTING

<110> OligoScience Biotechnology GmbH

<120> Microorganism for producing human milk oligosaccharide

<130> 198 377

<160> 42

<170> BiSSAP 1.3.6

<210> 1
<211> 30
<212> DNA
<213> Artificial Sequence

<220>
<223> FWD primer Pmgt

<400> 1
gtatactccg gagcaaacgc ctgaactccc                                        30

<210> 2
<211> 33
<212> DNA
<213> Artificial Sequence

<220>
<223> REV primer Pmgt

<400> 2
tctagaggaa gaataagtac gtgctatatt tag                                    33

<210> 3
<211> 27
<212> DNA
<213> Artificial Sequence

<220>
<223> FWD primer PmgtA

<400> 3
cgagctcctt tcgttattca gcacccg                                           27

<210> 4
<211> 27
<212> DNA
<213> Artificial Sequence

<220>
<223> REV primer PmgtA

<400> 4
cgagctcgcg atataatacc tgctggc                                           27

<210> 5
<211> 27
<212> DNA
<213> Artificial Sequence

<220>
<223> FWD primer mgtA 5'UTR

<400> 5
cgagctcctt tcgttattca gcacccg                                          27

<210> 6
<211> 27
<212> DNA
<213> Artificial Sequence

<220>
<223> REV primer mgtA 5'UTR

<400> 6
cgagctcaag gagtccctcc gcactgt                                          27

<210> 7
<211> 27
<212> DNA
<213> Artificial Sequence

<220>
<223> FWD primer SRRZ gene

<400> 7
ccgcatatgc cagaaaaaca tgacctg                                          27

<210> 8
<211> 30
<212> DNA
<213> Artificial Sequence

<220>
<223> REV primer SRRZ gene

<400> 8
ttagtcgacg caactctatc tgcactgctc                                       30

<210> 9
<211> 38
<212> DNA
<213> Artificial Sequence

<220>
<223> SRRz-SacI-fw

<400> 9

cgagctcaag gagatataat gccagaaaaa catgacct                                38

<210> 10
<211> 50
<212> DNA
<213> Artificial Sequence

<220>
<223> SRRz-luxTerm-PstI

<400> 10
cggctgcaga atctggcttt ttatattctc taagcgcgtg tgtattgctc                  50

<210> 11
<211> 29
<212> DNA
<213> Artificial Sequence

<220>
<223> SacI-PmgtA-UTR-fw

<400> 11
ctcgagctcc tttcgttatt cagcacccg                                         29

<210> 12
<211> 27
<212> DNA
<213> Artificial Sequence

<220>
<223> SacI-PmgtA-UTR-rv

<400> 12
cgagctcaag gagtccctcc gcactgt                                           27

<210> 13
<211> 29
<212> DNA
<213> Artificial Sequence

<220>
<223> Bsu36i-PmgtA-UTR-fw

<400> 13
cgcctcaggc tttcgttatt cagcacccg                                         29

<210> 14
<211> 53
<212> DNA
<213> Artificial Sequence

<220>
<223> SRRz-luxTerm-Bsu36i

<400> 14
cagcctgagg aaataataaa aaagccggat taataatctg gctttttata ttc          53

<210> 15
<211> 29
<212> DNA
<213> Artificial Sequence

<220>
<223> zwf-BglII-fw

<400> 15
gagagatcta tggcggtaac gcaaacagc                                     29

<210> 16
<211> 32
<212> DNA
<213> Artificial Sequence

<220>
<223> zwf-KpnI-rv

<400> 16
gcgggtacct tactcaaact cattccagga ac                                 32

<210> 17
<211> 33
<212> DNA
<213> Artificial Sequence

<220>
<223> gsk-KpnI-fw

<400> 17
gtaaaaggta ccatgaaatt tcccggtaaa cgt                                33

<210> 18
<211> 31
<212> DNA
<213> Artificial Sequence

<220>
<223> gsk_XhoI-rv

<400> 18
cgacctcgag ttaacgatcc cagtaagact c                                  31

<210> 19
<211> 26
<212> DNA
<213> Artificial Sequence

<220>
<223> rcsA-NdeI-fw

<400> 19
gtatcatatg tcaacgatta ttatgg                                            26


<210> 20
<211> 29
<212> DNA
<213> Artificial Sequence


<220>
<223> rcsA-BglII-rv

<400> 20
ccaagatcta tgtgttagcg catgttgac                                         29


<210> 21
<211> 34
<212> DNA
<213> Artificial Sequence


<220>
<223> lacY-BamHI-fw

<400> 21
gaaaggatcc atgtactatt taaaaaacac aaac                                    34


<210> 22
<211> 28
<212> DNA
<213> Artificial Sequence


<220>
<223> lacY-HindIII-rv

<400> 22
cattaagctt ttaagcgact tcattcac                                          28


<210> 23
<211> 35
<212> DNA
<213> Artificial Sequence


<220>
<223> lacY-S209I-fw

<400> 23
attcggcatt tattcttaag ctggcactgg aactg                                   35


<210> 24
<211> 30
<212> DNA
<213> Artificial Sequence

<220>
<223> lacY-S209I-rv

<400> 24
cttaagaata aatgccgaat ggttggcacc                                        30


<210> 25
<211> 35
<212> DNA
<213> Artificial Sequence


<220>
<223> trxA-BglII-fw

<400> 25
gagagatcta tgagcgataa aattattcac ctgac                                   35


<210> 26
<211> 30
<212> DNA
<213> Artificial Sequence


<220>
<223> trxA-KpnI-rv

<400> 26
cgaaggtacc attcccttac gccaggttag                                        30


<210> 27
<211> 31
<212> DNA
<213> Artificial Sequence


<220>
<223> manCB-NcoI-fw

<400> 27
cataccatgg cgcagtcgaa actctatcca g                                      31


<210> 28
<211> 30
<212> DNA
<213> Artificial Sequence


<220>
<223> manCB-HindIII-rv

<400> 28
cccaagcttt ggggtaaggg aagatccgac                                        30


<210> 29
<211> 30

<212> DNA
<213> Artificial Sequence

<220>
<223> gmd-NdeI-fw

<400> 29
cgccatatgt caaaagtcgc tctcatcacc                                        30

<210> 30
<211> 31
<212> DNA
<213> Artificial Sequence

<220>
<223> gmd-KpnI-rv

<400> 30
ggcggtacct tcctgacgta aaaacatcat t                                      31

<210> 31
<211> 29
<212> DNA
<213> Artificial Sequence

<220>
<223> pntAB-NcoI-fw

<400> 31
gggccatgga tgcgaattgg cataccaag                                         29

<210> 32
<211> 29
<212> DNA
<213> Artificial Sequence

<220>
<223> pntAB-BamHI-rv

<400> 32
ccgggatcct tacagagctt tcaggattg                                         29

<210> 33
<211> 70
<212> DNA
<213> Artificial Sequence

<220>
<223> lacIZYA-koF13

<400> 33
gtgaaaccag taacgttata cgatgtcgca gagtatgccg gtgtctctta gtgtaggctg      60

gagctgcttc                                                              70

<210> 34
<211> 70
<212> DNA
<213> Artificial Sequence

<220>
<223> lacIZYA-koR13

<400> 34
ttaaactgac gattcaactt tataatcttt gaaataatag tgcttatccc gatccgtcga 60

cctgcagttc 70

<210> 35
<211> 71
<212> DNA
<213> Artificial Sequence

<220>
<223> wcaJ-koF13

<400> 35
atgacaaatc taaaaaagcg cgagcgagcg aaaaccaatg catcgttaat cgtgtaggct 60

ggagctgctt c 71

<210> 36
<211> 70
<212> DNA
<213> Artificial Sequence

<220>
<223> wcaJ-koR13

<400> 36
tcaatatgcc gctttgttaa cgaagccctt gaataccgtc aggaaaacga gatccgtcga 60

cctgcagttc 70

<210> 37
<211> 70
<212> DNA
<213> Artificial Sequence

<220>
<223> lon-koF13

<400> 37
cagtcgtgtc atctgattac ctggcggaaa ttaaactaag agagagctct gtgtaggctg 60

gagctgcttc 70

<210> 38

<211> 70
<212> DNA
<213> Artificial Sequence

<220>
<223> lon-koR13

<400> 38
cgaattagcc tgccagccct gtttttatta gtgcattttg cgcgaggtca gatccgtcga 60

cctgcagttc 70

<210> 39
<211> 70
<212> DNA
<213> Artificial Sequence

<220>
<223> clpYQ-koF13

<400> 39
tacttttgta cggggtttgt actctgtatt cgtaaccaag gggtcagctc gtgtaggctg 60

gagctgcttc 70

<210> 40
<211> 70
<212> DNA
<213> Artificial Sequence

<220>
<223> clpYQ-koR13

<400> 40
gcctttcagc cccatcaaac aatgatgaaa atgattgaac gcgattatag gatccgtcga 60

cctgcagttc 70

<210> 41
<211> 70
<212> DNA
<213> Artificial Sequence

<220>
<223> trxB-koF13

<400> 41
atctcatggg cacgaccaaa cacagtaaac tgcttatcct gggttcaggc gtgtaggctg 60

gagctgcttc 70

<210> 42
<211> 70
<212> DNA
<213> Artificial Sequence

<220>
<223> trxB-koR13

<400> 42
gcatggtgtc gccttcttta cttttgttac tgatttgtaa aattattttg gatccgtcga      60

cctgcagttc                                                              70

## Description

### FIELD OF THE INVENTION

The present invention relates to genetically modified microorganisms for producing human milk oligosaccharide (HMO) and to a method of producing HMO using said microorganisms. The invention further relates to a medium, in which the microorganism of the invention has been cultured, to purified HMOs produced by the production method of the invention, and to uses of said medium and said HMOs.

### BACKGROUND

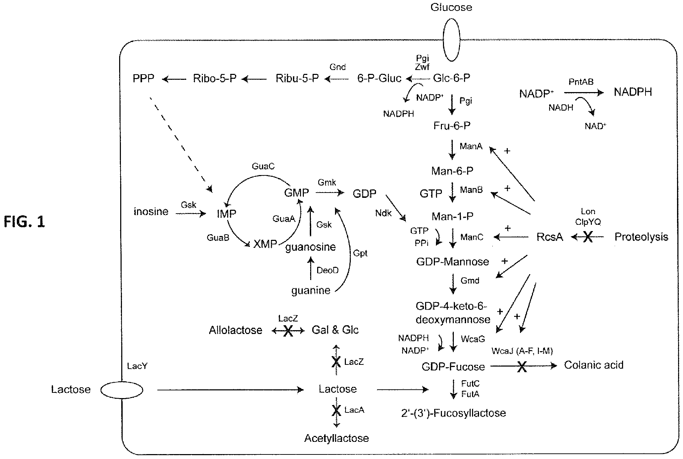

Human milk oligosaccharides (HMOs) are structurally diverse unconjugated glycans that are found in human breast milk. More than 200 HMOs have been identified to date, which are all built from only five monosaccharide building blocks, i.e. D-galactose (Gal), D-glucose (Glc), N-acetyl-D-glucosamine (GlcNAc), L-fucose (Fuc) and the sialic acid derivative N-acetyl-neuraminic acid, coupled to a lactose moiety (Petschacher and Nidetzky, J Biotechnol. 2016, 235:61-83). The reducing end can either be lactose or lactose extended by several disaccharide units of two structure types, which are lacto-N-biose (Gal-β1,3-GlcNAc, type I) or lactosamine (Gal-β1,4-GlcNAc, type II, LacNAc). Structures and classification of HMOs can be found e.g. in Fig. 1 and Table 1 of Petschacher and Nidetzky, 2016 (supra).

Among HMOs, 2'-Fucosyllactose (2-FL) is the most abundant and accounts for about 30% of all HMOs. Another prominent HMO is 3'-Fucosyllactose (3-FL). Further HMOs are lacto-N-tetraose, lacto-N-neotetraose and lacto-N-fucopentaose I. Besides such neutral HMOs, also acidic HMOs can be found in human milk, such as 3'-Sialyllactose, 6'-Sialyllactose and 3-fucosyl-3'-sialyllactose, disialyl-lacto-N-tetraose. HMOs are known to protect against pathogens such as *Campylobacter jejuni,* enteropathogenic *E. coli* and *Entamoeba histolytica* (Bode, Early Hum Dev. 2015; 91(11):619-22). 2-FL is regarded to have an ability to protect against infectious diseases by preventing epithelial level adhesions of toxins and pathogens. It stimulates the growth of certain Bifidobacteria and receptor analogons which provides protection to toxic and pathogenic challenge, which is most prevalent in infants. Based on such findings, HMOs, and especially 2-FL and 3-FL, are of particular interest for the use as functional ingredients in products ranging from infant nutrition over medical nutrition to functional foods and animal feed.

HMOs may be extracted from human breast milk or cow milk. However, in both cases it is difficult to obtain large amounts and/or to reach a satisfactory purity.

HMOs may also be chemically synthesized, however, the technology is not robust and the cost for chemically synthesized HMOs is high.

On the other hand, HMO production in genetically modified microorganisms holds promise to provide stable and safe means of production at low cost. US 7,521,212 describes a method for producing oligosaccharides by a genetically modified *E. coli* cell from the precursor lactose, involving the inactivation of the *lacZ* gene. EP 2 675 899, EP 2 877 574, EP 2 440 661 and EP 3 191 499 further describe different fucosyltransferases for producing 2-FL or 3-FL in genetically modified microorganisms. Huang *et al.,* 2017 describe the biosynthesis of 2-FL or 3-FL in genetically modified *Escherichia coli* (*E. coli*) and multiple modular optimization strategies thereto (Huang et al., Metab Eng. 2017; (41):23-38). EP 2 440 661 describes *E*. *coli* expressing sugar exporters to increase export of 2-FL into the medium.

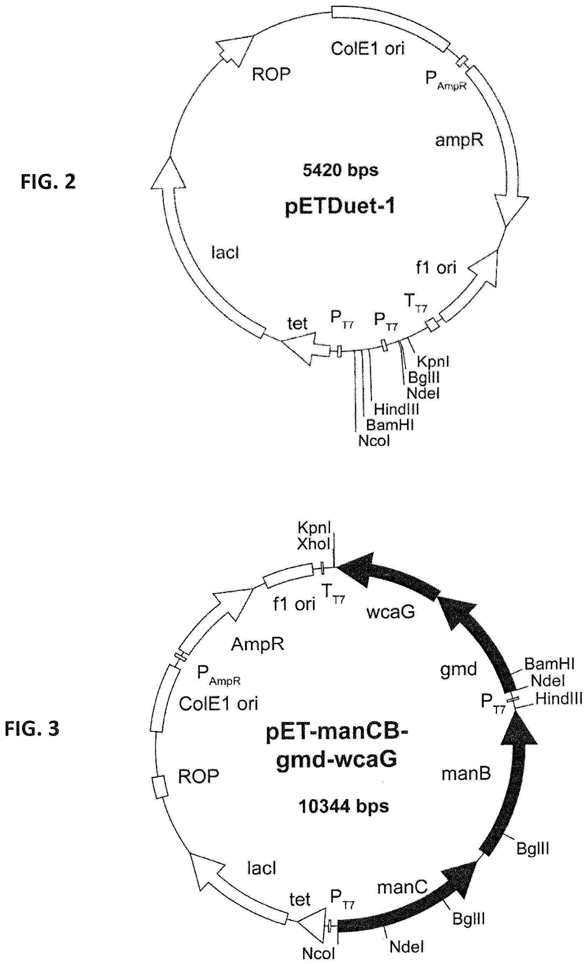

2-FL consists of lactose and fucose (Pereira & McDonald, Sci Rep. 2012; 84(1):637-655). The biosynthesis pathway of 2-(3-)-FL in *Escherichia coli* is shown schematically in Fig.1. *E. coli* is able to incorporate the precursors glucose and lactose into the cells via specific transporters. Glucose is absorbed via the phosphotransferase system and converted into glucose-6-phosphate. The latter serves as a source of carbon and energy to drive growth and as a precursor for GDP-L-fucose, an intermediate of the colanic acid biosynthesis. The *de novo* biosynthetic pathway of GDP-L-fucose is derived from the glycolytic intermediate fructose 6-phosphate and is based on the enzymes ManA (mannose 6-phosphate isomerase), ManB (phosphomanno-mutase), the GTP-dependent ManC (mannose 1-phosphate guanylyltransferase), Gmd (GDP-D-mannose 4,6-dehydratase) and the NADPH-dependent WcaG (GDP-L-fucose synthase), all derived from *E*. *coli* (FIG.2). The second precursor, lactose, is absorbed into the cells via the lactose permease LacY, a lactose/H⁺-symporter. The key enzyme for 2-FL production is the α-1,2-fucosyltransferase (FutC) derived from *Helicobacter pylori,* which transfers the fucose portion of the donor GDP-L-fucose to the acceptor lactose, producing 2-FL (FIG.2). By replacing the *futC* gene with the *futA* gene from *H. pylori,* which encodes an α-1,3-fucosyltransferase, the biosynthesis pathway can be used for the production of 3'-Fucosyllactose.

Despite recent advances, there is still a need for improved methods of biotechnological production of HMOs, and particularly 2-FL and/or 3-FL, in genetically modified microorganisms. Relying on the known 2-FL biosynthetic pathway in *E. coli,* the invention significantly improves the synthesis of HMOs and their release into the medium.

### BRIEF DESCRIPTION OF THE INVENTION

The present invention provides microorganisms for the improved production of HMOs and HMO production methods using the same.

In particular the present invention provides microorganisms comprising an inducible lysis system, which allows for the easy extraction of intracellular and extracellular HMOs at the same time without the need of additional processing steps to obtain the intracellular fraction. A preferred example of the inducible lysis system is an auto-inducible lysis system which is Mg²⁺-regulated, wherein the cells are lysed under Mg²⁺-depleted conditions, i.e. when the extracellular Mg²⁺ concentration is below a certain threshold value. Upon lysis of the cells, intracellular HMO may be released into the medium, in which the cells are cultured, leading to an increased HMO concentration in the medium. This may advantageously lead to increased yields of HMO as well as time and cost savings.

The invention also provides microorganisms comprising an exogenous gene encoding a lactose permease. The lactose permease is preferably a mutant of LacY, which is less prone to inhibition by intracellular lactose compared to wildtype LacY. This may advantageously lead to an increased yield of HMO.

The present invention further provides a method for producing HMOs using the microorganism of the invention.

Also provided is a medium, in which the microorganism of the invention has been cultured during the method of producing HMOs, and purified HMOs, obtained by the method for producing HMOs of the invention.

### Preferred embodiments:

1. A genetically modified microorganism for the production of human milk oligosaccharide.
2. The genetically modified microorganism according to item 1, wherein the microorganism is a bacterium.
3. The genetically modified microorganism according to item 1 or 2, wherein the microorganism is *Escherichia coli* (*E. coli*).
4. The genetically modified microorganism according to any one of items 1 to 3, wherein the microorganism comprises an inducible lysis system.
5. The genetically modified microorganism according to item 4, wherein the inducible lysis system is auto-inducible.
6. The genetically modified microorganism according to item 4 or 5, wherein the inducible lysis system is Mg²⁺-regulated.
7. The genetically modified microorganism according to any one of items 4 to 6, wherein the inducible lysis system is regulated by the exogenous free Mg²⁺ concentration.
8. The genetically modified microorganism according to any one of items 4 to 7, wherein the microorganism comprises a Mg²⁺-regulated promoter.
9. The genetically modified microorganism according to item 8, wherein the Mg²⁺-regulated promoter is the Pmgt promoter from the *mgtB* gene of *Salmonella typhimurium* or the PmgtA promoter of *Escherichia coli.*
10. The genetically modified microorganism according to item 8 or 9, wherein the microorganism further comprises an additional Mg²⁺-regulated element, preferably the 5'UTR of the *mgtA* gene of *Escherichia coli.*
11. The genetically modified microorganism according to any one of items 4 to 10, wherein the microorganism comprises a lysis genes.
12. The genetically modified microorganism according to item 11, wherein the lysis gene is a lysis gene from a bacteriophage.
13. The genetically modified microorganism according to item 12, wherein the bacteriophage is λ bacteriophage.
14. The genetically modified microorganism according to item 13, wherein the lysis genes are the λ bacteriophage lysis genes SRRz or the A bacteriophage mutant Sam7 lysis genes.
15. The genetically modified microorganism according to any one of items 11 to 14, wherein the lysis genes are inducibly expressed under free Mg²⁺-depleted conditions.
16. The genetically modified microorganism according to any one of items 4 to 15, wherein the microorganism comprises a Mg²⁺-regulated promoter and lysis genes, wherein expression of the lysis gene(s) is controlled by the Mg²⁺-regulated promoter, and optionally by an additional Mg²⁺-regulated element.
17. The genetically modified microorganism according any one of items 1 to 16, wherein the microorganism is unable to cleave lactose into glucose and galactose.
18. The genetically modified microorganism according to item 1 to 17, wherein the *lac* operon is inactivated, preferably by deletion.
19. The genetically modified microorganism according to any one of items 1 to 18, wherein the microorganism comprises an exogenous gene encoding a lactose permease.
20. The genetically modified microorganism according to item 19, wherein the lactose permease is the *E. coli* LacY protein.
21. The genetically modified microorganism according to item 19 or 20, wherein the lactose permease has A198V and/or S209I mutations.
22. The genetically modified microorganism according to any one of items 1 to 20, wherein the human milk oligosaccharide is 2'-Fucosyllactose and/or 3'-Fucosyllactose.
23. The genetically modified microorganism according to any one of items 1 to 22, wherein the microorganism comprises an exogenous gene encoding a fucosyltransferase.
24. The genetically modified microorganism according to item 23, wherein the fucosyltransferase is an α-1,2-fucosyltransferase and/or an α-1,3-fucosyltransferase.
25. The genetically modified microorganism according to item 23 or 24, wherein the gene encoding a fucosyltransferase is a heterologous gene and/or wherein the gene encoding a fucosyltransferase is driven by a heterologous promoter.
26. The genetically modified microorganism according to any one of items 23 to 25, wherein the gene encoding a fucosyltransferase is codon-optimized for expression in the microorganism.
27. The genetically modified microorganism according to any one of items 23 to 26, wherein the gene encoding a fucosyltransferase is *futC* from *Helicobacter pylori* or *futA* from *Helicobacter pylori.*
28. The genetically modified microorganism according to any one of items 1 to 27, wherein the microorganism comprises a heterologous gene encoding a chaperone.
29. The genetically modified microorganism according to item 28, wherein the chaperone is human Hsp70.
30. The genetically modified microorganism according to any one of items 1 to 29, wherein the microorganism overexpresses a transcriptional regulatory protein, which increases the expression of genes involved in GDP-L-fucose *de novo* synthesis.
31. The genetically modified microorganism according to any one of items 1 to 30, wherein the *rcsA* gene is overexpressed.
32. The genetically modified microorganism according to item 30 or 31, wherein the *rcsA* gene is derived from *E. coli.*
33. The genetically modified microorganism according to any one of items 1 to 32, wherein a gene encoding a Lon protease family protein is inactivated, preferably by deletion.
34. The genetically modified microorganism according to item 33, wherein the Lon protease family protein is *E. coli* Lon protease.
35. The genetically modified microorganism according to any one of items 1 to 34, wherein the *wcaJ* gene is inactivated, preferably by deletion.
36. The genetically modified microorganism according to any one of items 1 to 35, wherein the *zwf* gene and the two *pntAB* genes are overexpressed.
37. The genetically modified microorganism according to item 36, wherein the *zwf* gene and the *pntAB* genes are derived from *E. coli.*
38. The genetically modified microorganism according to any one of items 1 to 37, wherein the *gsk* gene is overexpressed.
39. The genetically modified microorganism according to item 38, wherein the *gsk* gene is derived from *E. coli.*
40. The genetically modified microorganism according to any one of items 1 to 39, wherein the *trxA* gene is overexpressed.
41. The genetically modified microorganism according to item 39, wherein the *trxA* gene is derived from *E*. *coli.*
42. The genetically modified microorganism according to any one of items 1 to 41, wherein the *trxB* gene is inactivated, preferably by deletion.
43. A method for producing human milk oligosaccharide, which comprises:
   (a) culturing the genetically modified microorganism according to any one of items 1 to 42 in a medium.
44. The method for producing human milk oligosaccharide according to item 43, wherein the medium further comprises lactose.
45. The method for producing human milk oligosaccharide according to item 43 or 44, wherein the medium further comprises free Mg²⁺.
46. The method for producing human milk oligosaccharide according to item 45, wherein the free Mg²⁺ concentration in the medium before the beginning of culturing is 5 mM or more.
47. The method for producing human milk oligosaccharide according to item 45 or 46, wherein the free Mg²⁺ concentration in the medium at the end of culturing is 10 µM or less.
48. The method for producing human milk oligosaccharide according to any one of items 43 to 47, wherein the culturing is conducted in a batch culture, optionally a fed-batch culture.
49. The method for producing human milk oligosaccharide according to any one of items 43 to 48, wherein the culture volume is 10L or more, preferably 100L or more.
50. The method for producing human milk oligosaccharide according to any one of items 43 to 49, wherein the concentration of human milk oligosaccharide in the medium at the end of the culture is at least 15 g/L, preferably at least 20 g/L.
51. The method for producing human milk oligosaccharide according to any one of items 43 to 50, wherein the human milk oligosaccharide is 2'-Fucosyllactose and/or 3'-Fucosyllactose.
52. The method for producing human milk oligosaccharide according to any one of items 43 to 51, which further comprises:
   (b) purifying the human milk oligosaccharide from the culture medium and/or from the microorganism itself.
53. The method for producing human milk oligosaccharide according to item 52, wherein the human milk oligosaccharide is purified from the culture medium after lysis has been induced via the inducible lysis system.
54. The method for producing human milk oligosaccharide according to item 52 or 53, wherein the human milk oligosaccharide is purified from the culture medium when the concentration of free Mg²⁺ in the medium is 10 µM or less and/or when the lysis can be directly visualized.
55. The method for producing human milk oligosaccharide according to item 54, wherein the lysis can be directly visualized by a drop in OD₆₀₀ values of the culture.
56. Medium obtainable by the method according to any one of items 43 to 51.
57. Human milk oligosaccharide obtainable by the method according to any one of items 52 to 55.
58. Use of the medium according to item 56 or of the human milk oligosaccharide according to item 57 for the preparation of animal feed.

### BRIEF DESCRIPTION OF THE DRAWINGS

**Fig.1****:** Biosynthesis of 2'-(3')-Fucosyllactose in *Escherichia coli.* Glc-6-P: glucose 6-phosphate; Fru-6-P: fructose 6-phosphate; Man-6-P: mannose 6-phosphate; 6-P-Gluc: 6-phospho-gluconate; Ribu-5-P: ribolusoe-5-phosphate; Ribo-5-P: ribose-5-phosphate; PPP: phosphoribosyl-pyrophosphate; gal: galactose; glc: glucose; XMP: xanthosine 5'-monophosphate; IMP: inosine-5-monophosphate; Pgi: phosphoglucose-isomerase; ManA: mannose 6-phosphate isomerase; ManB: phosphomanno mutase; ManC: α-D-mannose 1-phosphate guanylyltransferase; Gmd: GDP-mannose 6-dehydrogenase; WcaG: GDP-L-fucose synthase; WcaJ: UDP-glucose carrier transferase; Wca A-F, I-M: colanic acid biosynthesis genes; LacA: β-galactosid-transacetylase; LacZ: β-galactosidase; LacY: lactose permease; Lon: protease; ClpYQ (HsIUV): protease; RscA: positive transcription regulator; guaA: GMP-synthetase; GuaB: IMP-Dehydrogenase; GuaC: GMP-reductase; DeoD: purine nucleotide phosphorylase; Gpt: guanine phosphoribosyltransferase; Gnd: 6-phosphogluconate dehydrogenase; Ndk: nucleotide diphosphate-kinase.
**Fig.2****:** pETDuet-1. *E. coli* expression vector with double T7-promoters (P_{T7}), T7-terminator (T_{T7}), ColE1 ori, ampicillin resistance gene (ampR), lac repressor (*lacI*).
**Fig.3****:** pET-manCB-gmd-wcaG. *E*. *coli* pETDuet-1 with double T7-promoters (P_{T7}), T7-terminator (T_{T7}), pBR322 ori, ampicillin resistance gene (*ampR*) and lac repressor (*lacI*), *manB:* phosphomanno-mutase, *manC:* α-D-mannose 1-phosphate guanylyl transferase, *gmd:* GDP-mannose 6-dehydrogenase; *wcaG:* GDP-L-fucose synthase.
**Fig.4****:** pCDFDuet-1. *E. coli* expression vector with double T7-promoters (P_{T7}), T7-terminator (T_{T7}), CloDF13 ori, streptomycin resistance gene (*SmR*), lac repressor (*lacI*).
**Fig.5****:** pUC19. *E*. *coli* cloning vector with ampicillin resistance gene *(AmpR),* lacZ alpha peptide (*lacZ*), ori.
**FIG.6****:** pCDF-futC-lacY*-hHSP70-trxA-SRRz. *E. coli* pCDFDuet-1 double T7-promoters (P_{T7}), T7-terminator (T_{T7}), CloDF13 ori, streptomycin resistance gene (*SmR*) and lac repressor (*lacI*). *futC:* alpha-1,2-fucosyltransferase, *lacY**: lactose permease with S2091, *hHSP70:* human heat shock protein 70, *trxA*: thioredoxin, *SRRz*: lysis genes from bacteriophage lambda under control of *mtgA*-promoter wit 5'-UTR and *3'- LuxICDABEG* terminator.
**FIG.7****:** pCOLADuet-1. *E*. *coli* expression vector with double T7-promoters (P_{T7}), T7-terminator (T_{T7}), ColA ori, kanamycin resistance gene (KmR) and lac repressor (*lacI*).
**FIG.8****:** pCOLA-pntAB-rcsA-zwf-gsk. *E*. *coli* pCOLADuet-1 double T7-promoters (P_{T7}), T7-terminator (T_{T7}), ColA ori, kanamycin resistance gene (*KmR*) and lac repressor (*lacI*). *pntAB:* membrane-bound transhydrogenase, *rcsA*: positive transcription regulator of colanic acid biosynthesis, *zwf*: Glucos 6-phosphate dehydrogenase, *gsk*: guanosine inosine kinase.

### DETAILED DESCRIPTION OF THE INVENTION

### Definitions and general techniques

Unless otherwise defined, all technical and scientific terms used herein have the same meaning as commonly understood by one of ordinary skill in the art to which this invention belongs.

Terms like "comprises" or "comprising" are used such that the terms may optionally be replaced by the terms "consists of" and "consisting of', respectively.

Terms like "include" or "including" are used such that the terms may optionally be replaced by the terms "consists of" and "consisting of", respectively.

The term "genetically modified" refers to any modification which has been introduced into a cell with respect to the genetic material of said cell. This may include the introduction of new genetic material, deletion of genetic material, mutation of genetic material etc.

The term "inducible lysis system" means that cell lysis may be induced by external stimuli. "Auto-inducible" means that the external stimuli do not have to be actively applied by the skilled person carrying out the invention.

The terms "external stimulus" or "external stimuli" refers to a chemical or physical stimulus or stimuli which is/are imposed on a cell from the outside.

The term "Mg²⁺-regulated" means that something is regulated by the presence or absence of free magnesium (Mg²⁺) ions. The "exogenous free Mg²⁺ concentration" refers to the concentration of free Mg²⁺ ions acting on the cells from the outside, e.g. from within the medium surrounding the cells. "Mg²⁺-depleted conditions" refer to a low free Mg²⁺ concentration in the medium or a complete absence of free Mg²⁺ in the medium.

A "lysis gene" is a gene encoding a factor, e.g. a protein, which causes cell lysis when expressed in a cell. The term "inducibly expressed" means that a gene is not constitutively expressed but, instead, expression may be induced by external stimuli.

The term "the lysis genes are controlled by the Mg²⁺-regulated promoter" means that the genes are expressed or not expressed when the promoter is active or inactive, respectively, due to the absence or presence of free Mg²⁺ ions.

"Free Mg²⁺ ions" are those dissolved in e.g. the culture medium, which are not e.g. complexed by a chelating agent, such as EDTA. Thus, addition of e.g. a chelating agent may be used to reduce the concentration of free Mg²⁺ in the medium. If not stated otherwise, "Mg²⁺" refers to "free Mg²⁺ ions" herein.

The term "exogenous gene" refers to a gene which is introduced into a cell from the outside, e.g. by transformation. However, the term "exogenous gene" does not dictate the origin of said gene, e.g. an exogenous gene in *E*. *coli* may be derived from *E*. *coli* or from another organism. An exogenous gene may be episomal, for example present in an (expression) vector, such as a plasmid, or it may be integrated into a chromosome of the cell.

The term "heterologous gene" refers to a gene which is introduced into a cell from the outside and is derived from different organism than the cell into which it is introduced, e.g. a heterologous gene in *E*. *coli* is not derived from *E*. *coli.* A heterologous gene may be episomal, for example present in an (expression) vector, such as a plasmid, or it may be integrated into a chromosome of the cell.

A "heterologous promoter" when operably linked to a gene, refers to a promoter which is not naturally associated with the gene.

Exogenous or heterologous genetic material may be introduced into the microorganism by techniques commonly known in the art. For example, genetic material may be introduced into bacteria by transformation. Transformation may be conducted e.g. by heat-shock of chemically competent bacteria or by electroporation of electrocompetent bacteria (see e.g. Hanahan et al., Methods Enzymol. 1991; 204:63-113; Miller and Nickoloff, Methods Mol Biol. 1995; 47:105-13).

Exogenous or heterologous genetic material may be integrated into a chromosome of a cell by techniques commonly known in the art, such as the lambda Red system-mediated integration or CRISPR/Cas9 (see e.g. Juhas and Ajioka, Microb Cell Fact. 2016; 15(1):172; Reisch and Prather, Sci Rep. 2015; 5:15096).

A gene which is derived from a particular organism, such as *E*. *coli,* may also include mutants obtained by introducing mutations in the original sequence from said organism, or it may also include codon-optimized variants.

The term "codon-optimized for expression in the microorganism" means that the nucleic acid sequence of the open-reading frame (ORF) of a gene is optimized for translation into a protein in the organism into which the gene is introduced.

Where the microorganism of the invention is defined to comprise a gene encoding a particular protein, it is intended that the microorganism may also express the gene to produce the encoded protein.

The terms "overexpressed" or "overexpression" in the context of genes mean that the expression level of a gene in a microorganism, in which the gene is overexpressed, are higher than the expression level of the same gene in the same microorganism which does not overexpress said gene. Genes may be overexpressed by techniques commonly known in the art. For example, additional exogenous gene copies may be introduced into a cell, a cell may be treated with an exogenous transcriptional activator, or an exogenous gene encoding a transcriptional activator may be introduced into the cell. Introduction of additional gene copies is preferred in the present invention.

"Inactivation" of a gene means that the gene does not express any functional protein anymore. This includes almost complete or complete lack of expression or expression of dysfunctional proteins. An almost complete or complete lack of expression may be achieved for example via deletion of control sequences, such as the promoter, deletion of (parts of) the open reading frame (ORF) of the gene or deletion of the entire gene including promoter and ORF. A knockout of the gene is thus also encompassed by the term. Expression of dysfunctional proteins may be achieved for example by partial deletion, point mutations and/or insertions in/of the ORF, leading to expression e.g. of truncated and/or catalytically inactive proteins. Methods for introducing deletions, point mutations and/or insertions are well-known in the art. See for example, Reisch and Prather, 2015 (supra) or Zerbini et al. (Microb Cell Fact. 2017; 16(1):68), Jensen et al. (Sci Rep. 2015; 5:17874).

A particular nucleotide sequence, for example a sequence underlying a particular gene of interest or a promoter sequence etc., can either be amplified by polymerase chain reaction from the genomic sequences of an organism, e.g. *E*. *coli,* or it can be chemically synthesized by methods commonly known in the art.

GenBank Accession numbers refer to the unique identifiers used in GenBank, accessible e.g. via https://www.ncbi.nlm.nih.gov/genbank/. Gene identifiers (GI) refer to the unique numbers used to identify a particular gene, and may be queried e.g. at https://www.ncbi.nlm.nih.gov/gene/.

### Embodiments

### Genetically modified microorganism for the production of HMOs

The present invention provides genetically modified microorganisms for the production of HMOs.

The HMOs to be produced in the invention are based on the disaccharides lactose or N-acetyllactosamine. HMOs based on lactose are preferred.

Preferred human milk oligosaccharides in the context of the invention include, but are not limited to, 2'-Fucoyllactose (2-FL), 3'-Fucosyllactose (3-FL), 3'-Sialyllactose (3-SL), 6'-Sialyllactose (6-SL), lacto-N-tetraose (LNT), lacto-N-neotetraose (LNnT), lacto-N-hexaose (LNH), iso-lacto-N-octaose, iso-lacto-N-neooctaose, para-lacto-N-octaose, lacto-N-fucopentaose I (LNFP I), lacto-N-fucopentaose II (LNFP II), lacto-N-fucopentaose III (LNFP III), lacto-N-fucopentaose V (LNFP V), LS-tetrasaccharide a (LST a), LS-zetrasaccharide b (LST b), LS-zetrasaooharide c (LST c) and disialyllacto-N-tetraose (DSLNT).

The trisaccharides 2-FL and/or 3-FL are preferably produced in the present invention, and 2-FL is particularly preferred. The structure of 2-FL and 3-FL is shown in (Pereira & McDonald, Sci Rep. 2012; 84(1):637-655).

The invention is described in more detail concerning the manipulation of genes and pathways within the enterobacterium *Escherichia coli* K-12 (*E*. *coli*) with regard to the production of 2-FL and/or 3-FL. However, the invention is similarly applicable to production of other HMOs in *E*. *coli* or microorganisms other than *E*. *coli.* Microbiological production of HMOs other than 2-FL and/or 3-FL has been described previously. For example, Priem *et al.* showed that lactose can be glycosylated to arrive at the trisaccharide GlcNAc-β1,3-Gal-β1,4-Glc, using expression of the β1,3-N-acetylglucosaminyltransferase LgtA (e.g. GI: 904226) from *Neisseria meningitides,* which can be further extended to lacto-N-neotetraose (LNnT) by the β1,4-galactosyltransferase LgtB (e.g. GI: 904227), also from *N. meningitides* (Priem et al., Glycobiology. 2002; 12(4):235-40). Subsequent intracellular fucosylation of LNnT, catalyzed by *H. pylori* β1,3-fucosyltransferase FutA or FutB, can provide a HMO mixture with lacto-N-neodifucohexaose II (LNnDFHII) as the main product (Dumon et al., Biotechnol Prog. 2004; 20(2):412-9). Different production methods are further discussed by Han *et al.* (Han et al., 2012, Biotechnol Adv. 2012; 30(6):1268-78).

The microorganism to be used in the present invention is not particularly limited. It is preferably a microorganism, which either has the natural ability of producing an HMO or is able to generate precursor molecules for production of an HMO intracellularly or to import such precursors from the extracellular space. Examples of the microorganism include a bacterium or a yeast. Examples of a bacterium include *Escherichia coli, Erwinia herbicola (Pantoea agglomerans), Citrobacfer freundii, Pantoea citrea, Pectobacterium carotovorum,* or *Xanthomonas campestris.* Bacteria of the genus *Bacillus* may also be used, including *Bacillus subtilis, Bacillus licheniformis, Bacillus coagulans, Bacillus thermophilus, Bacillus laterosporus, Bacillus megaterium, Bacillus mycoides, Bacillus pumilus, Bacillus lentus, Bacillus cereus,* and *Bacillus circulans.* Similarly, bacteria of the genera *Lactobacillus* and *Lactococcus* may be modified using the methods of this invention, including but not limited to *Lactobacillus acidophilus, Lactobacillus salivarius, Lactobacillus plantarum, Lactobacillus helveticus, Lactobacillus delbrueckii, Lactobacillus rhamnosus, Lactobacillus bulgaricus, Lactobacillus crispatus, Lactobacillus gasseri, Lactobacillus casei, Lactobacillus reuteri, Lactobacillus jensenii,* and *Lactococcus lactis. Streptococcus thermophiles* and *Proprionibacterium freudenreichii* are also suitable bacterial species for the present invention. Also included as part of this invention are strains, modified as described here, from the genera *Enterococcus* (e.g., *Enterococcus faecium* and *Enterococcus thermophiles*), *Bifidobacterium* (e.g., *Bifidobacterium longum, Bifidobacterium infantis,* and *Bifidobacterium bifidum*), *Sporolactobacillus* spp., *Micromomospora* spp., *Micrococcus* spp., *Rhodococcus* spp., and *Pseudomonas* (e.g., *Pseudomonas fluorescens* and *Pseudomonas aeruginosa).* Examples of a yeast include *Saccharomyces cerevisiae, Kluyveromyces lactis* and *Pichia pastoris.* The microorganism is preferably a bacterium, and *E*. *coli* is particularly preferred. The *E*. *coli* strain to be used is not particularly limited.

The release of HMOs from bacterial cells is an essential process for industrial production. About 50% of 2-FL yield remains in the cells while the remainder is discharged into the medium (Huang *et al.,* 2017). Heat-induced lysis at 100°C is required to gain access to the total 2-FL amount. To improve the recovery process, an autolysis system is established in the microorganism of the invention. The microorganism of the invention may thus comprise an inducible lysis system. The type of lysis system is not particularly limited as long as it can induce lysis due to an external stimulus. For example, the lysis genes of bacteriophage lambda (*S*, *R* and *Rz*) may be under control of the Mg²⁺-repressible P*_{mgtA}*-UTR promoter (Tamekou Lacmata et al., Bioengineered. 2017; 26:1-6)). When free Mg²⁺ is consumed (e.g. at 10 µM or less), synthesis of the lysis genes is induced, facilitating the release of 2-FL into the medium by degradation of the cell wall component peptidoglycan. There are several alternatives available for the establishment of an autolysis system, which are based on an Mg²⁺-regulated promoter as described by Zhang et al. (J Microbiol Methods. 2009; 79(2):199-204), a nickel-inducible system as described by Liu and Curtiss, (Proc Natl Acad Sci USA. 2009; 106(51):21550-4), a thermosensitive lambda repressor (Juhas et al., PLoS One. 2016; 11(10):e0165778), a nisin-inducible promoter (Visweswaran et al., Appl Microbiol Biotechnol. 2017 Feb;101(3):1099-1110), an arabinose-inducible promoter (Masuda et al., FEMS Microbiol Lett. 2016; 363(6)), an arabinose-inducible promoter, wherein MgSO₄ counteracts the system (Li et al., Curr Microbiol. 2016; 72(4):390-6) or lysis induction by addition of solvents (Hajnal et al., Appl Microbiol Biotechnol. 2016; 100(21):9103-9110). These systems are also summarized in Table 1.

**TABLE 1 Inducible autolysis systems**

| **CHEMICAL or PHYSICAL STIMULI** | **SPECIES** | **REFERENCE** |
|---|---|---|
| Mg²⁺-repression | *E. coli* | (Zhang et *al*., 2009; Tamekou Lacmata *et al.,* 2017) |
| Nickel-induction | *E. coli* | (Liu and Curtiss, 2009) |
| heat-induction | *E. coli* | (Juhas *et al.,* 2016) |
| Nisin-induction | *Lactococcus* | (Visweswaran *et al.,* 2017) |
| L-Arabinose-induction | *E. coli* | (Masuda *et al.,* 2016) (Li *et al.,* 2016) |
| induction by organic solvents (e.g. chloroform, isopropanol) | *E. coli* | (Hajnal *et al.,* 2016) |

The Mg²⁺-regulatable system has the advantage that cell lysis can be promoted by the addition of e.g. Ethylenediaminetetraacetic acid (EDTA), a chelating agent that sequesters metal ions (such as Ca²⁺ or Mg²⁺). Moreover, Mg²⁺ as a divalent metal ion is required for α-1,2- and α-1,3-fucosyltransferase activities (Zhang et al., Glycobiology. 2010; 20(9):1077-88; Zhao et al., Chem Commun (Camb). 2016; 52(20):3899-902). Thus, in the presence of Mg²⁺, 2'-Fucosyllactose synthesis may be improved due to enhanced enzyme activity, while in its absence cell lysis is induced to facilitate product recovery via cell wall disruption. Accordingly, the Mg²⁺-regulated system is preferably used in the invention and preferably, the Mg²⁺-regulated system is Mg²⁺-repressible.

For the inducible lysis system a Mg²⁺-regulated promoter may be used. The Mg²⁺-regulated promoter is preferably regulated by the exogenous free Mg²⁺ concentration. It is preferably active under Mg²⁺-depleted conditions. Mg²⁺-depleted conditions are conditions wherein the growth medium comprises 500 µM or less, 100 µM or less, 50 µM or less, 10 µM or less, 1 µM or less or 0 µM of free Mg²⁺. Preferably, the Mg²⁺-depleted conditions are 10 µM or less of free Mg²⁺. The Mg²⁺-regulated promoter is preferably the *PmgtB* promoter from the *mgtB* gene (e.g. GI: 1250998) of *Salmonella typhimurium,* the P*pagC* promoter from the *pagC* gene (e.g. 1248241) of *S*. *tyhphimurium* or the *PmgtA* promoter from the *mgtA* gene (e.g. GI: 948778) of *E*. *coli.*

The PmgtB promoter from the *S*. *typhimurium mgtB* gene is as described in e.g. Zhang *et al.,* 2009 (supra) and is obtainable e.g. by polymerase chain reaction (PCR) using the *S*. *typhimurium* genome as template and using the primers 5'-GTATACTCCGGAGCAAACGCCTGAACTCCC-3' (SEQ ID NO: 1) and 5'-TCTAGAGGAAGAATAAGTACGTGCTATATTTAG-3' (SEQ ID NO: 2). The PmgtA promoter from *E*. *coli* is as described in e.g. Tamekou Lacmata *et al.,* 2017 (supra) and is obtainable e.g. by polymerase chain reaction (PCR) using the *E*. *coli* genome as template and using the primers 5'-CGAGCTCCTTTCGTTATTCAGCACCCG-3' (SEQ ID NO: 3) and 5'-CGAGCTCGCGATATAATACCTGCTGGC-3' (SEQ ID NO: 4).

The Mg²⁺-regulated promoter may be combined with additional Mg²⁺-regulated elements, such as the 5'UTR of the *mgtA* gene of *E*. *coli.* The 5'UTR of the *mgtA* gene from *E*. *coli* is as described in e.g. Tamekou Lacmata *et al.,* 2017 (supra) and the PmgtA promoter together with the *mgtA* 5'UTR is obtainable e.g. by polymerase chain reaction (PCR) using the *E*. *coli* genome as template and using the primers 5'-CGAGCTCCTTTCGTTATTCAGCACCCG-3' (SEQ ID NO: 3) and 5'-CGAGCTCAAGGAGTCCCTCCGCACTGT-3' (SEQ ID NO: 5).

The microorganism comprising an inducible lysis system may further comprise one or more lysis gene(s). The lysis gene is not particularly limited as long as it can lyse a cell when it is expressed therein. For example, the lysis gene may be from a bacteriophage, such as from A bacteriophage, MS2 bacteriopage, ΦX174 bacteriophage or RLT bacteriophage. Examples of lysis genes include *ydfD* from *E*. *coli* as described by Masuda *et al.,* 2016 (supra), *sicG* from *Streptococcus dysgalactiase* subsp. *equisimilis, vanX* from *E. coli,* SRRz from *Salmonella* phage P22, *yncE* from *S*. *enterica serovar Paratyphi A,* lysis genes from *Staphylococcus aureus* phage P68.

Preferred examples of lysis gene are the SRRz gene from A bacteriophage (e.g. as found in the sequence of GenBank Accession number: NC_001416) or the Sam7 mutant thereof, carrying a G to A mutation at position 161 in the S gene, the MS2 phage lysis gene encoding the E protein or the ΦX174 bacteriophage lysis gene encoding the L protein. A SRRz gene is as described in e.g. Zhang *et al.,* 2009 (supra) and is obtainable e.g. by PCR using λ bacteriophage genome as template and primers 5'-CCGCATATGCCAGAAAAACATGACCTG-3' (SEQ ID NO: 6) and 5'-TTAGTCGACGCAACTCTATCTGCACTGCTC-3' (SEQ ID NO: 7). MS2 bacteriopage or ΦX174 bacteriophage lysis genes are as described in e.g. Juhas *et al.,* 2016 (supra).

The microorganism may also comprise two or more lysis genes, either of the same type or different. Optionally, a combination of two or more different lysis genes may be used, since a different mechanism of action may lead to more efficient lysis. For example, the microorganism may comprise a lysis gene from A and MS2 bacteriophages, from A and ΦX174 bacteriophages, from MS2 and ΦX174 bacteriophages or from A, MS2 and ΦX174 bacteriophages. However, the invention is not limited to these combination and other lysis genes as described may be used alone or in combination. Preferably, the two or more lysis genes are located in tandem and are controlled by the same promoter and the same optional additional regulatory elements in a polycistronic manner.

The one or more lysis gene(s) may be inducibly expressed under Mg²⁺-depleted conditions and is/are preferably controlled by the Mg²⁺-regulated promoter with or without an additional regulatory element. If the 5'UTR of the *mgtA* gene is used as additional regulatory element, the expression cassette of the lysis gene(s) preferably comprise(s) the Mg²⁺-regulated promoter followed by the 5'UTR of the *mgtA* gene, followed by the ORF (or ORFs) of the lysis gene(s).

The expression cassette which may be used for inducible lysis as described above may be present in the microorganism as an episomal vector, such as an expression vector, e.g. a plasmid, or integrated into a host chromosome.

Furthermore, as the precursor for HMOs is lactose, it is desirable for the intracellular production of HMOs to achieve high intracellular levels of lactose. Therefore, the microorganism of the invention may be unable to cleave lactose into glucose and galactose. This may be achieved by inactivating the *lacZ* gene encoding the β-galactosidase LacZ responsible for lactose cleavage, or inactivating the entire lac operon, which encodes LacZ as well as the lactose repressor LacI, the transacetylase LacA and the lactose permease LacY. Inactivation may be achieved by inhibitors of expression, by introducing inactivating mutations or by deletion. Inactivation of the whole *lac* operon, comprising the genes *lacI, lacZ, lacY* and *lacA,* may increase the intracellular lactose availability by preventing i) the degradation of lactose into glucose and galactose and ii) by preventing the formation of allo- or acetyl-lactose. The latter additionally simplifies the purification process by eliminating two contaminating sugar molecules.

A lactose permease is responsible for uptake of lactose into the cell. Many bacteria have the inherent ability of transporting lactose from the culture medium into the cell, by using a transport protein that is either a homolog of the *E*. *coli* lactose permease (e.g., as found in *Bacillus licheniformis*)*,* or a member of the PTS sugar transport family (e.g., as found in *Lactobacillus casei* and *Lactobacillus rhamnosus*)*.* Accordingly, introducing an exogenous lactose permease gene, such as *E. coli* LacY, may increase the intracellular lactose levels. In the case of bacteria which do not have an inherent ability of transporting extracellular lactose into the cell (including those having the entire lac operon inactivated), this ability may be conferred or restored by introduction of an exogenous lactose permease gene, such as *E. coli lacY.* The microorganism of the invention may thus comprise an exogenous gene encoding a lactose permease. It is preferred that the lactose permease is *E. coli* LacY (e.g. GI: 949083). It is particularly preferred that the *E*. *coli* LacY has A198V and/or S209I mutations (Wilson et al., Biochim Biophys Acta. 1990; 1029(1):113-6). Both mutations are predicted to prevent the interaction between LacY and its inhibitor protein Enzyme-IIa^{Glc} (Hogema et al., Mol Microbiol. 1999; 31(6):1825-33). As a result, the autoregulation mechanism may be suppressed at high intracellular lactose concentrations. Thus, the yield of HMOs may be enhanced by increasing the lactose uptake. Moreover, as lactose is a natural inducer of the T7 promoter expression system (see below), the expression e.g. of the enzymes required for 2-FL synthesis may be further improved by increased intracellular lactose availability.

Preferably, the microorganism of the invention is for the production of 2-FL and/or 3-FL.

Exemplary genetic modifications for high production of 2-FL in *E*. *coli* is shown in Table 2.

**TABLE 2 Genetic modifications for high production of 2'-fucosyllactose in E. coli**

| **Nr**. | **GENE** | **ENZYME** | FUNCTION |
|---|---|---|---|
| **Overexpression** | | | |
| **1** | *manB* | phosphomanno mutase | GDP-L-fucose biosynthesis |
| **2** | *manC* | mannose 1-phosphate guanylyltransferase | |
| **3** | *gmd* | GDP-D-mannose 4,6-dehydratase | |
| **4** | *wcaG* | NADPH-dependent GDP-L-fucose synthase | |
| **5** | *rcsA* | positive transcription regulator | positive activator of colanic acid biosynthesis genes |
| **6** | *gsk* | guanosine-inosine kinase | GTP-synthesis required for ManC |
| **7** | *lacY* | lactose permease | LacY-S209I or A198V with increased lactose uptake |
| **8** | *futC* | α-1,2-fucosyltransferase (codon optimized for *E*. *coli*) | Transfer of GDP-L-fucose to lactose |
| **9** | *zwf* | G6PDH, glucose 6-phosphate dehydrogenase | NADPH-regeneration required for WcaG |
| **10** | *pntAB* | membrane bound transhydrogenase | |
| **11** | *trxA* | thioredoxin | Increased expression and solubility of recombinant proteins (Redox activity promotes disulphide bond formation, putative chaperone function, processivity factor of T7 DNA polymerase) |
| **12** | *hHsp70* | human heat shock protein 70 (codon optimized for *E. coli*) | chaperone function and establishment of a self-inducible expression system that foregoes IPTG addition for induction |
| **13** | *SRR_{z}* | λ bacteriophage lysis gene under control of PmgtA-UTR and bidirectional *LuxICDABEG* terminator from *Vibrio fischeri* | autolysis system to improve product release, lysis genes under control of Mg²⁺-repressible promoter PmgtA-UTR from *E*. *coli* |

| **Deletion** | | | |
|---|---|---|---|
| **1** | *ΔlacIZYA* | Lacl repressor, LacZ β-galactosidase, LacY permease and LacA transacetylase | knockout blocks hydrolysis of lactose and its conversion to allo- or acetyllactose |
| **2** | *Δlon* | Lon-Protease | Knockout prevents RcsA degradation |
| **3** | *ΔclpYQ* | ClpYQ protease (synonym HsIUV) | |
| **5** | *ΔwcaJ* | UDP-glucose lipid carrier transferase | Knockout prevents conversion of GDP-L-fucose to colanic acid instead of 2-fucosyllactose |
| **5** | *ΔtrxB* | NADPH-dependent thioredoxin reductase | knockout blocks consumption of NADPH required for WcaG and increases protein solubility by promoting disulphide bond formation |

2-FL or 3-FL can be generated by coupling the activated sugar GDP-L-fucose to lactose. As described above, *E*. *coli* is capable of importing the precursor molecule lactose into the cells via the lactose permease LacY (a lactose/H⁺ symporter). Furthermore, *E*. *coli* is capable of importing glucose into the cell via the phosphotransferase system. Glucose may serve as energy source to promote growth and as a precursor for the production of GDP-L-fucose, which is a natural intermediate of colanic acid biosynthesis, via a de *novo* pathway (see Fig.1). The *de novo* pathway for the biosynthesis of the activated sugar GDP-L-fucose diverges from the glycolysis intermediate fructose-6-phosphate and is based on the *E. coli* derived enzymes ManA and ManB, GTP-dependent ManC, NADPH-dependent Gmd and WcaG. Alternatively, GDP-L-fucose may also be generated by the salvage pathway from fucose which is imported into the cell.

The fucose moiety of GDP-L-fucose can be transferred to the acceptor lactose in the cell via a fucosyltransferase (EC 2.4.1.x), thereby generating 2-FL or 3-FL (see Fig.1). Key enzymes for the generation of 2-FL are α-1,2-fucosyltransferases, such as FutC from *Helicobacter pylori.* For the generation of 3-FL, α-1,3-fucosyltransferases, such as FutA from *Helicobacter pylori,* may be used. Accordingly, the microorganism of the invention also comprises an exogenous gene encoding a fucosyltransferase, preferably a α-1,2-fucosyltransferase and/or an α-1,3-fucosyltransferase. The gene encoding a fucosyltransferase is preferably a heterologous gene and/or driven by a heterologous promoter.

Several fucosyltransferases have been identified in bacteria such as *Bacteroides fragilis, B. vulgatus, Campylobacter jejuni, E. coli, Helicobacter bilis, H. hepaticus, H. mustelae, H. pylori* and *Thermosynechococcus elongatus.* Because many organisms have differential codon usages, the presence of codons with limited availability of cognate tRNA anticodons can lower heterologous gene expression due to ribosome stalling. To guarantee a high protein expression in the microorganism of the invention, the codon usage of the α-1,2- and α-1,3-fucosyltransferases encoding genes (e.g. *futC* and *futA*) may be optimized for the microorganism of the invention. Accordingly, the gene encoding a fucosyltransferase may be codon-optimized for expression in the microorganism. Codon-optimization may result in increased intracellular fucosyltransferase protein levels which may improve the yield of 2-FL and/or 3-FL.

Preferred examples of α-1,2-fucosyltransferase for use in the invention are FutC from *Helicobacter pylori* (e.g. NCBI accession no. KY499613), FutL from *H. mustelae* (e.g. NCBI accession no. WP_013023529.1), FutF from *H. bilis* (e.g. NCBI accession no. WP_020995676.1), WcfB from *Bacillus fragilis* NCTC 9343 (e.g. NCBI accession no. WP_005817145.1), *WbsJ* from *E. coli* 0128 (e.g. NCBI accession no. AAO37698.1), WbwK from *E. coli* 086 (e.g. NCBI accession no. AAO37719.1), WbgL from *E*. *coli* 0126 (e.g. NCBI accession no. ADN43847.1), WbiQ from *E. coli* O127 (e.g. NCBI accession no. CAS09719.1), FutG from *Campylobacter jejuni* (e.g. NCBI accession no. WP_002861859.1), FutN from *Bacteroides vulgatus* ATCC 8482 (e.g. NCBI accession no. ALK85429.1), WcfB and WcfW from *Bacteroides fragilis* NCTC9343 (e.g. NCBI accession no. WP_005817145.1 and WP_005813010.1).

Preferred examples of α-1,3-fucosyltransferase for use in the invention are FutA from *Helicobacfer pylori* (e.g. NCBI accession no. AAD07447.1), FutB from H. pylori 26695 (e.g. NCBI accession no. AAD07710.1), FutD from *H. trogontum* (e.g. NCBI accession no. WP_052089242.1), FutE from *H. bilis* (e.g. NCBI accession no. WP_034580283.1), FutH from *H. typhlonius* (e.g. NCBI accession no. WP_052082154.1), FutJ (Hh0072) and FutK (Hh1776) from *H. hepaticus* (e.g. NCBI accession no. WP_011114915.1 and AAP78373.1), FutM from *B. fragilis* (e.g. NCBI accession No. CAH09151.1.1).

In a preferred embodiment, the activity of a fucosyltransferase is increased by the presence of Mg²⁺.

Fucosyltransferases may form insoluble intracellular *inclusion bodies,* which may reduce the availability of active fucosyltransferases in the cell. The presence of an exogenously introduced chaperone may advantageously influence the solubility of the fucosyltransferase and thereby improve production of 2-FL and/or 3-FL. The microorganism of the invention may thus further comprise an exogenous gene encoding a chaperone. In a preferred embodiment, the chaperone is human Hsp70 (e.g. GenBank Accession Number: BC112963). Apart from chaperon function, human Hsp70 may further be utilized to establish a self-inducible expression system (Briand et al., Sci Rep. 2016; 6:33037), which may be used to avoid the addition of the expression inducer IPTG in the culture medium. As IPTG is also a substrate for the lactose permease, omission of IPTG may increase lactose uptake and thereby further improve the production of HMOs, particularly 2-FL and/or 3-FL.

Apart from the above modifications, a number of other genes may be introduced into or inactivated in the cells in order to further optimize production of HMOs, particularly of 2-FL and/or 3-FL. RcsA is a positive transcriptional regulator of colanic acid biosynthesis in *E*. *coli,* which uses GDP-L-fucose as intermediate. An overexpression of *rcsA* (e.g. GenBank Accession Number M58003) increases the transcription of the genes involved in biosynthesis of the GDP-L-fucose intermediate (see Fig.1), which may improve 2-FL and/or 3-FL synthesis by strengthening the GDP-L-fucose pathway (Huang *et al.,* 2017, supra). Thus, the microorganism of the invention may also overexpress the *rcsA* gene, which is preferably derived from *E*. *coli.*

However, RcsA is under the control of the Lon- and ClpYQ proteases. Thus, the respective *lon* and *clpYQ-*genes may be inactivated in order to prevent a down-regulation of the required GDP-L-fucose biosynthesis genes. Overexpression of *rcsA* in combination with a *lon*/*clpYQ* inactivation may dramatically improve GDP-L-fucose and thus 2-FL production. Accordingly, in the microorganism of the invention a Lon protease family gene, preferably *E*. *coli* Lon protease (e.g. GenBank Accession Number L20572), and/or the *clpYQ* genes (NC_000913.3) may be inactivated.

Furthermore, the *wcaJ* gene (e.g. GenBank Accession Number (amino acid) BAA15900) codes for an UDP-glucose lipid carrier transferase predicted to initiate the colanic acid biosynthesis. Inactivation of the *wcaJ* gene may be performed in order to i) prevent the consumption of GDP-L-fucose by the competitive colanic acid pathway, ii) prevent high viscosity of the culture medium due to export of the exopolysaccharide colanic acid and iii) simplify the 2-FL purification process by eliminating another contaminating sugar molecule (colanic acid). Thus, in the microorganism of the invention the *wcaJ* gene, preferably the *E*. *coli wcaJ* gene, may be inactivated.

The co-factors NADPH and GTP also play an important role in GDP-L-fucose biosynthesis, whose availability may limit the synthesis of the activated sugar (GDP-L-fucose) and thereby also the total yield of HMOs, such as 2-FL and/or 3-FL. To increase the availability of co-factors for GDP-L-fucose synthesis, four genes may be overexpressed in the microorganism of the invention. Zwf is part of the pentose phosphate pathway, while PntA and PntB belong to the transhydrogenase system. Overexpression of *zwf* and *pntAB* may significantly contribute to NADPH-regeneration, thereby providing the required co-factor for the NADPH-dependent WcaG enzyme involved in GDP-L-fucose synthesis (Fig.1). Thus, the microorganism of the invention may co-overexpress the *zwf* gene and both *pntAB* genes (KEGG Entry, *E*. *coli* K-12 MG1655: b1852, b1603 and b1602), which are preferably derived from *E*. *coli.*

The second co-factor, GTP, is synthesized via two alternative routes, the *de novo* GTP biosynthesis pathway via the formation of phosphoribosyl pyrophosphate or the salvage pathway from exogenous delivered guanosine. In both cases, an adequate supply of GMP can increase the GTP level and thus improve the efficiency of GDP-L-fucose production. The overexpression of the *gsk* gene (guanosine inosine kinase, e.g. GI: 946584), involved in both metabolic pathways, in the microorganism of the invention may increase the GTP-availability required for ManC (Fig.1). As a result, the GDP-L-fucose synthesis and, consequently, the HMO production may be dramatically improved (Lee et al., Appl Microbiol Biotechnol. 2012; 93(6):2327-34). Accordingly, the microorganism of the invention may overexpress the *gsk* gene, which is preferably derived from *E*. *coli.*

Primers for cloning of exemplary genes discussed above may be found e.g. in Table S2 of Huang *et al.,* 2017 (supra).

Additional optimization may involve manipulation of thioredoxin activity. The *trxA* gene (KEGG Entry, *E*. *coli* K-12 MG1655: b3781) codes for the major physiological electron donor in *E*. *coli.* Overexpression of thioredoxin (TrxA) is known to improve heterologous protein expression by promoting the formation of disulphide bond formation in poorly soluble proteins. However, two other functions have also been linked to TrxA, a potential chaperone function and a function as a processivity factor of the T7-DNA polymerase (Weickert et al., Curr Opin Biotechnol. 1996; 7(5):494-9). Thus, overexpression of *trxA* may be performed to i) increase the solubility of enzymes needed for HMO synthesis and ii) positively influence the expression of the respective genes. Accordingly, the microorganism of the invention may overexpress the thioredoxin *trxA* gene, which is preferably derived from *E*. *coli.*

Furthermore, the *trxB* gene (KEGG Entry, *E. coli* BL21 (DE3): ECD_00892) codes for a NADPH-dependent thioredoxin reductase (TrxB) that regenerates TrxA from its oxidized form. Inactivation of *trxB* limits the reducing potential in the cytoplasm and allows the formation of disulfide bonds (Weickert *et al.,* 1996, supra). Inactivation of *trxB* may be performed to i) improve the protein solubility and enzyme activity and ii) prevent the exhaustion of NADPH, the co-factor required for WcaG (Fig.1). Accordingly, in the microorganism of the invention the *trxB* gene may be inactivated.

### Method for producing HMOs

The present invention also provides a method for producing HMOs using the genetically modified microorganism of the invention.

By culturing the microorganism of the invention in a medium, HMOs may be produced.

The culture medium is not particularly limited as long as it supports the growth of the microorganism. Examples of a medium for culturing the microorganism of the invention are LB medium (10 g/L tryptone; 5 g/L yeast extract; 10 g/L NaCl, pH 7.5), modified M9 minimal medium containing glucose (or glycerol) as carbon source (36 g/L glucose; 12.8 g/L Na₂HPO₄·7H₂O, 3 g/L KH₂PO₄, 2 g/L NH₄Cl, 0.5 g/L NaCl, 0.25 g/L MgSO₄·7H₂O, 14.7 mg/L CaCl₂·2H₂O, 10 mg/L thiamine, 2 g/L yeast extract, 0.1%(v/v) Triton-X 100, and 1 mL/L stock trace metal solution; stock trace metal solution: 25 g/L FeCl₃·6H₂O, 2 g/L CaCl₂·2H₂O, 2 g/L ZnCl₂, 2 g/L Na₂MoO₄·2H₂O, 1.9 g/L CuSO₄·5H₂O, and 0.5 g/L H₃BO₃, pH 7.2) (see Huang *et al.,* 2017, supra), minimal medium containing 2.68 g/L (NH₄)₂SO₄, 1g/L (NH₄)₂-H-citrate, 26.42 g/L glycerol, 14.6 g/L K₂HPO₄, 0.241 g/L MgSO₄, 2g/L Na₂SO₄, 4 g/L NaH₂PO₄ · H2O, 0.5 g/L NH₄Cl, 10 mg/L thiamine and 3 ml/L trace element solution (0.5 g/L CaCl₂ · 2 H₂O, 16,7 g/L FeCl₃ · 6 H₂O, 20.1 g/L Na₂-EDTA, 0.18 g/L ZnSO₄ · 7 H₂O,0.1 g/L MnSO₄ · H₂O, 0.16 g/L CuSO₄ · 5 H₂O and 0.18 g/L CoCl₂ · 6 H₂O) (Baumgärtner *et al.,* Microb Cell Fact. 2013; 12:40), or minimal medium containing 20 g/L glycerol, 13.5 g/L KH₂PO₄, 4.0 g/L (NH₄)₂HPO₄, 1.7 g/L citric acid, 1.4 g/L MgSO₄·7H₂O, 10 ml/L trace element solution (10 g/L Fe(III) citrate, 2.25 g/L ZnSO₄·7H₂O, 1.0 g/L CuSO₄·5H₂O, 0.35 g/L MnSO₄·H₂O, 0.23 g/L Na₂B₄O₇·10H₂O, 0.11 g/L (NH₄)₆Mo₇O₂₄, 2.0 g/L CaCl₂·2H₂O (Chin et al., J Biotechnol. 2016. pii: S0168-1656(16)31632-7).

The microorganism of the invention may be cultured at any temperature suitable for growth, e.g. at a temperature from 16 °C to 37 °C, such as from 16 °C to 30 °C, and preferably from 16 °C to 25 °C.

In a preferred embodiment, the medium comprises lactose. The lactose concentration in the medium before the beginning of the culture may be 20 g/L or more, 12 g/L or more, 10 g/L or more, 8 g/L or more, between 8 and 20 g/L, between 10 and 20 g/L, or between 12 and 20 g/L. Preferably the lactose concentration in the medium before the beginning of the culture is between 10 and 20 g/L.

In another preferred embodiment, the medium comprises free Mg²⁺.

The free Mg²⁺ concentration (i.e. the concentration of free Mg²⁺) in the medium before beginning of the culture may be 80 mM or more, 70 mM or more, 60 mM or more, 50 mM or more, 40 mM or more, 30 mM or more, 20 mM or more, 10 mM or more, 5 mM or more, 1 mM or more, between 1 and 50 mM, between 5 and 50 mM, between 10 and 50 mM, between 20 and 50 mM, between 1 and 10 mM, between 5 and 10 mM, between 1 and 5 mM, or between around 50 and 60 µM. Preferably the free Mg²⁺ concentration in the medium before beginning of the culture is between 5 and 20 mM.

The free Mg²⁺ concentration at the end of the culture may be 500 µM or less, 100 µM or less, 50 µM or less, 10 µM or less, 1 µM or less, or 0 µM. Preferably, the free Mg²⁺ concentration at the end of the culture is 10 µM or less.

Preferably, the Mg²⁺ is added to the medium in the form of MgSO₄.

At the end of the culture, a chelating agent, such as EDTA or EGTA, may optionally be added, preferably in sufficient amounts to sequester residual free Mg²⁺ ions and trigger lysis.

The method for producing HMOs of the invention is preferably conducted in a batch culture, or a fed-batch culture. The batch culture is a closed system culture in which cells are grown in a fixed volume of nutrient culture medium under specific environmental conditions without addition of further nutrients. In a fed-batch culture, nutrients are supplied to the bioreactor during cultivation. In both cases, the cells and products remain in the bioreactor until the end of the culture.

The culture volume in the method for producing HMOs of the invention is preferably 10L or more, 100L or more, 1000L or more, 10000L or more, 50000L or more, between 10L and 100L, between 100L and 1000L, between 1000L and 10000L or between 10000L or more than 50000L. Preferably the culture volume is between 100L and 1000L.

The concentration of HMOs in the medium at the end of the culture is preferably at least 15 g/L, more preferably at least 20 g/L, even more preferably at least 30 g/L, and most preferably at least 50 g/L.

In the invention, a single HMO may be produced or two or more HMOs may be produced at the same time, preferably by a single genetically modified microorganism. For example, expression of a α-1,2-fucosyltransferase and a α-1,3-fucosyltransferase in the same cell may lead to the production of 2-FL, 3-FL and/or difucosylated structures such as lactodifucotetraose or lacto-*N*-difucohexaose.

The HMO produced in the present invention is preferably 2-FL and/or 3-FL, and 2-FL is particularly preferred.

The HMO may be retrieved from the medium and/or from the microorganism itself. In order to retrieve the HMO from the microorganism, the microorganism has to be disrupted. Cell disruption by cell lysis may be conducted by chemical or physical means as commonly known in the art, or lysis may be induced by an inducible lysis system as described herein.

The method for producing HMOs of the invention may further comprise the step of purifying the HMO from the culture medium. Purifying in this context means that the HMO is present in pure form or essentially pure form after purification. After purification, the HMO, e.g., 2-FL and/or 3FL, preferably constitutes at least 90%, 95%, 98%, 99%, or 100% (w/w) of the purified product with respect to the dry weight. Purification may be done by techniques well known to the person skilled in the art. For example, HMO may be purified from the medium by methods commonly known to the person skilled in the art, e.g. by column chromatography using a charcoal step and elution with 35-50% ethanol, by an ethanol gradient or by size exclusion.

Purity can be assessed by any known method, such as thin layer chromatography or other electrophoretic or chromatographic techniques commonly known in the art.

Preferably, the HMO is purified from the culture medium after lysis has been induced via an inducible lysis system. Upon lysis of the cells, intracellular HMO is released into the medium leading to an increased HMO concentration in the medium. This may lead to increased yields of HMO as well as time and cost savings due to said increased yield and fewer processing steps. Accordingly, the HMO is preferably purified from the culture medium when the concentration of free Mg²⁺ in the medium is 10 µM or less and/or when the lysis can be directly visualized. Lysis can be directly visualized for example by a visual clearing of the culture and/or by measuring the OD₆₀₀ absorbance values of the culture at 600nm (optical density). OD₆₀₀ values of the culture may be measured by a method commonly known to the person skilled in the art, e.g. using a spectrophotometer. A drop in OD₆₀₀ values indicates that lysis of cells has taken place. Accordingly, in the invention, lysis can be directly visualized by a drop in OD₆₀₀ values of the culture. The invention further provides a medium obtainable by the method for producing HMOs of the invention. Due to the production of HMOs by culturing the microorganism of the invention, said medium contains HMOs. The concentration of HMOs in the medium at the end of culturing is preferably at least 15 g/L, more preferably at least 20 g/L, even more preferably at least 30 g/L, and most preferably at least 50 g/L. The medium of the invention may further be dehydrated, for example by lyophilisation.

The invention also provides purified HMOs obtainable by the method of producing HMOs of the present invention.

The medium and the purified HMOs of the present invention may be used for the preparation of products for the consumption by humans, such as infant nutrition, and/or animals. The medium and the purified HMOs of the present invention may be used for the preparation of animal feed. The animal feed may be for companion animals (dogs, cats), livestock (bovine, equine, ovine, caprine, or porcine animals, as well as poultry) and/or fish. It is preferable that the animal feed is for cattle. Preferably the animal feed is a milk replacement product for calves.

The present invention will be illustrated by the following non-limiting examples.

### EXAMPLES

### Material and Methods

The strains, plasmids and primers used are listed in Table 3 and 4. *E*. *coli* BL21 (DE3) is used as host strain for HMO production, *E*. *coli* TOP10 for plasmid construction and maintenance. *E*. *coli* is cultured in LB medium (10 gL⁻¹ tryptone, 5 gL⁻¹ yeast extract, 10 gL⁻¹ NaCl) supplemented with ampicillin (100 µg ml⁻¹), streptomycin (50 µg ml⁻¹) or kanamycin (50 µg ml⁻¹) when required. For gene deletions, the lambda Red recombinase/flippase system from Jensen et al. 2015 (Sci Rep 2015; 5:17874) may be used. Vector pSIJ8 is transformed into competent cells of *E*. *coli* BL21 (DE3). The lambda Red genes are induced in the presence of 15 mM L-arabinose. The kanamycin-resistance cassette (kanR) is amplified from vector pKD13 (Datsenko and Wanner, 2000), which is flanked by 50 bp long homologous target sequences and FRT sites, and transformed into L-arabinose induced competent cells. After deletion, removal of the antibiotic cassettes is achieved by induction of the flippase recombinase in the presences of 50 mM L-rhamnose. For curing of the temperature-sensitive helper plasmid (pSIJ8), cells are grown at 42°C. For gene expression, the vectors pETDuet-1, pCDFDuet-1 and pCOLADuet-1 may be used (Table 3, Fig.2, 4 and 7). Plasmids are transformed into competent cells of *E*. *coli* BL21 (DE3). For batch fermentation, *E*. *coli* is grown in 100 ml LB with 36 g/L glucose. When OD₆₀₀ 0,6-0,8 is reached, 0.1 mM IPTG or 0.2% lactose is added for induction. After 2 and 10 h of growth at 16-25 °C, 5 g/L lactose is added for HMO-production.

**TABLE 3 Strains and plasmids.**

| **STRAINS or PLASMIDS** | **GENOTYPE** | **ORIGIN** |
|---|---|---|
| **STRAINS** | | |
| *E. coli* K-12 MG1655 | F-λ-*ilvG-frb-50rph-1* | CGSC |
| *E. coli* BL21 (DE3) | F- *ompT hsdSB* (rB- mB-) *gal dcm rne131* A(DE3) | Merckmillipor e |
| *E. coli* TOP10 | *F-mcrA Δ*(*mrr-hsdRMS-mcrBC*) φ80*lacZ*ΔM15Δ*lacX74 recA1 araD139* Δ(*ara-leu*)7697 *galU galK rpsL* (*StrR*) *endA1 nupG* | Thermofisher |

| **PLASMIDS** | | |
|---|---|---|
| pETDuet-1 | Double T7 promoters; ColE1 ori AmpR | Novagen |
| pCDFDuet-1 | Double T7 promoters; CloDF13 ori StrR | Novagen |
| pCOLADuet-1 | Double T7 promoters; ColA ori KanR | Novagen |
| pET-manCB-gmd- *wcaG* | pETDuet-1 carrying *manC-manB* (Ncol, Hindlll) and *gmd-wcaG* (NdeI, KpnI) from *E*. *coli* K-12 MG1655 | |
| pCDF-futC-lacY*- hHSP70-trxA-SRRz | pCDFDuet-1 carrying human codon optimized *hHSP70* (Ndel, BglII), *lacY**(S209I BamHI, Hindlll) from *E*. *coli* K12-MG1655, codon optimized *futC* from *Helicobacter pylori* with N-terminal 3x Asp-tag (NcoI, BamHI), *trxA* (BglII, KpnI) from *E*. *coli* K12-MG1655, SRRz under control of PmgtA-UTR from *E*. *coli* BL21 (DE3) and LuxICDABEG-terminator from *Vibrio fischeri* | |
| pCOLA-pntAB-rcsA-zwf-gsK | pCOLADuet-1 carrying *rcsA* (NdeI, BglII) and *zwf* (BglII, Kpn) from *E. coli* K12-MG1655, gsk (KpnI, XhoI) from *E. coli* K12-ATCC10798, *pntAB* (Ncol, BamHI) from *E*. *coli* K12-MG1655 | |
| pKD13 | *AmpR,* FRT-*KmR*-FRT, oriR6K (ts) | addgene |
| pSIJ8 | *AmpR, araC-P*_{araB}::*gam-bet-exo-*tL3, *rhaR-rhaS,* p_{rhaB}::*flp*-tL3, Rep ori1, repA101 (ts) | addgene |

**TABLE 4. Primers used for gene overexpression or deletion.**

| **PRIMER** | **SEQUENCE** |
|---|---|
| **OVEREXPRESSION** | |
| **PmgtA-UTR_SRRz-luxTerm** | |
| SRRz-SacI-fw | CGAGCTCAAGGAGATATAATGCCAGAAAAACATGACCT |
| SRRz-luxTerm-PstI | CGGCTGCAGAATCTGGCTTTTTATATTCTCTAAGCGCGTGTGTATTGCTC |
| SacI-PmgtA-UTR-fw | CTCGAGCTCCTTTCGTTATTCAGCACCCG |
| SacI-PmgtA-UTR-rv | CGAGCTCAAGGAGTCCCTCCGCACTGT |
| Bsu36i-PmgtA-UTR-fw | CGCCTCAGGCTTTCGTTATTCAGCACCCG |
| SRRz-luxTerm-Bsu36i | CAGCCTGAGGAAATAATAAAAAAGCCGGATTAATAATCTGGCTTTTTATATT C |
| ***zwf*** | |
| zwf-BglII-fw | GAGAGATCTATGGCGGTAACGCAAACAGC |
| zwf-KpnI-rv | GCGGGTACCTTACTCAAACTCATTCCAGGAAC |
| ***gsk*** | |
| gsk-KpnI-fw | GTAAAAGGTACCATGAAATTTCCCGGTAAACGT |
| gsk_XhoI-rv | CGACCTCGAGTTAACGATCCCAGTAAGACTC |
| ***rcsA*** | |
| rcsA-NdeI-fw | GTATCATATGTCAACGATTATTATGG |
| rcsA-BgIII-rv | CCAAGATCTATGTGTTAGCGCATGTTGAC |
| ***lacY*** | |
| lacY-BamHI-fw | GAAAGGATCCATGTACTATTTAAAAAACACAAAC |
| lacY-HindIII-rv | CATTAAGCTTTTAAGCGACTTCATTCAC |
| lacY-S209I-fw | ATTCGGCATTTATTCTTAAGCTGGCACTGGAACTG |
| lacY-S209I-rv | CTTAAGAATAAATGCCGAATGGTTGGCACC |
| ***trxA*** | |
| trxA-BgIII-fw | GAGAGATCTATGAGCGATAAAATTATTCACCTGAC |
| trxA-KpnI-rv | CGAAGGTACCATTCCCTTACGCCAGGTTAG |
| ***manC-manB*** | |
| manCB-NcoI-fw | CATACCATGGCGCAGTCGAAACTCTATCCAG |
| manCB-HindIII-rv | CCCAAGCTTTGGGGTAAGGGAAGATCCGAC |
| ***gmd-wcaG*** | |
| gmd-Ndel-fw | CGCCATATGTCAAAAGTCGCTCTCATCACC |
| gmd-KpnI-rv | GGCGGTACCTTCCTGACGTAAAAACATCATT |
| ***pntA-pnt8*** | |
| pntAB-Ncol-fw | GGGCCATGGATGCGAATTGGCATACCAAG |
| pntAB-BamHI-rv | CCGGGATCCTTACAGAGCTTTCAGGATTG |
| **KNOCKOUT** | |
| lacIZYA-koF13 | GTGAAACCAGTAACGTTATACGATGTCGCAGAGTATGCCGGTGTCTCTTA GTGTAGGCTGGAGCTGCTTC |
| lacIZYA-koR13 | TTAAACTGACGATTCAACTTTATAATCTTTGAAATAATAGTGCTTATCCC GATCCGTCGACCTGCAGTTC |
| wcaJ-koF13 | ATGACAAATCTAAAAAAGCGCGAGCGAGCGAAAACCAATGCATCGTTAATC GTGTAGGCTGGAGCTGCTTC |
| wcaJ-koR13 | TCAATATGCCGCTTTGTTAACGAAGCCCTTGAATACCGTCAGGAAAACGA GATCCGTCGACCTGCAGTTC |
| lon-koF13 | CAGTCGTGTCATCTGATTACCTGGCGGAAATTAAACTAAGAGAGAGCTCT GTGTAGGCTGGAGCTGCTTC |
| lon-koR13 | CGAATTAGCCTGCCAGCCCTGTTTTTATTAGTGCATTTTGCGCGAGGTCA GATCCGTCGACCTGCAGTTC |
| clpYQ-koF13 | TACTTTTGTACGGGGTTTGTACTCTGTATTCGTAACCAAGGGGTCAGCTC GTGTAGGCTGGAGCTGCTTC |
| clpYQ-koR13 | GCCTTTCAGCCCCATCAAACAATGATGAAAATGATTGAACGCGATTATAG GATCCGTCGACCTGCAGTTC |
| trxB-koF13 | ATCTCATGGGCACGACCAAACACAGTAAACTGCTTATCCTGGGTTCAGGC GTGTAGGCTGGAGCTGCTTC |
| trxB-koR13 | GCATGGTGTCGCCTTCTTTACTTTTGTTACTGATTTGTAAAATTATTTTG GATCCGTCGACCTGCAGTTC |

| | |
|---|---|
| underlined: restriction sites (overexpression), vector binding site (knockout) | |

### Construction of expression vectors:

### pET-manCB-gmd-wcaG

The genes *manC-manB* and *gmd-wcaG* are amplified from the genomic DNA of *E*. *coli* K-12 MG1655 using the primers listed in Table 4. First, the *gmd-wcaG* PCR product is digested with NdeI/KpnI and inserted into the likewise cut vector pETDuet-1 (Fig.2), yielding pET-gmd-wcaG. Next, the *manC-manB* PCR fragment is digested with NcoI/HindII and inserted into the similar cut vector pET-gmd-wcaG to generate vector pET-manCB-gmd-wcaG (Fig.3). Selected clones are grown in LB with ampicillin (100 µg ml⁻¹) and verified by restriction mapping and DNA sequencing.

### pCDF-futC-lacY*-hHSP70-trxA-SRRz

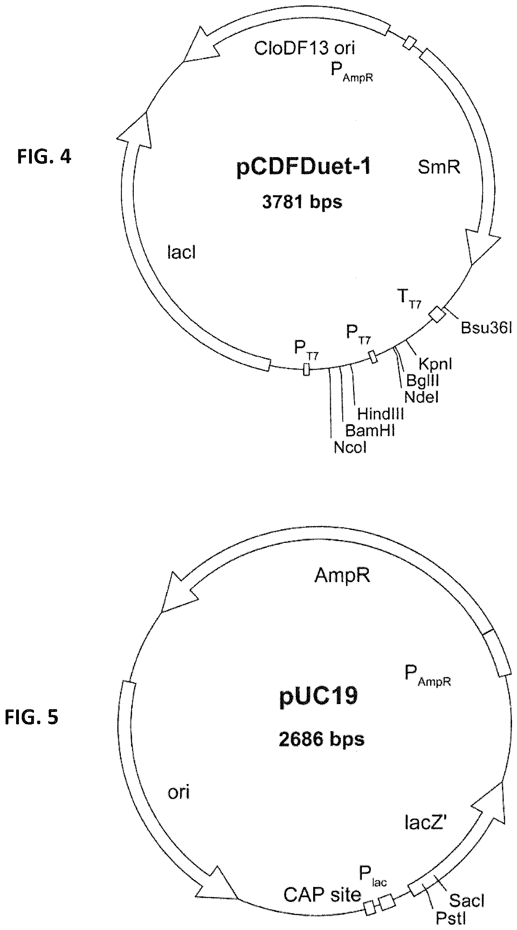

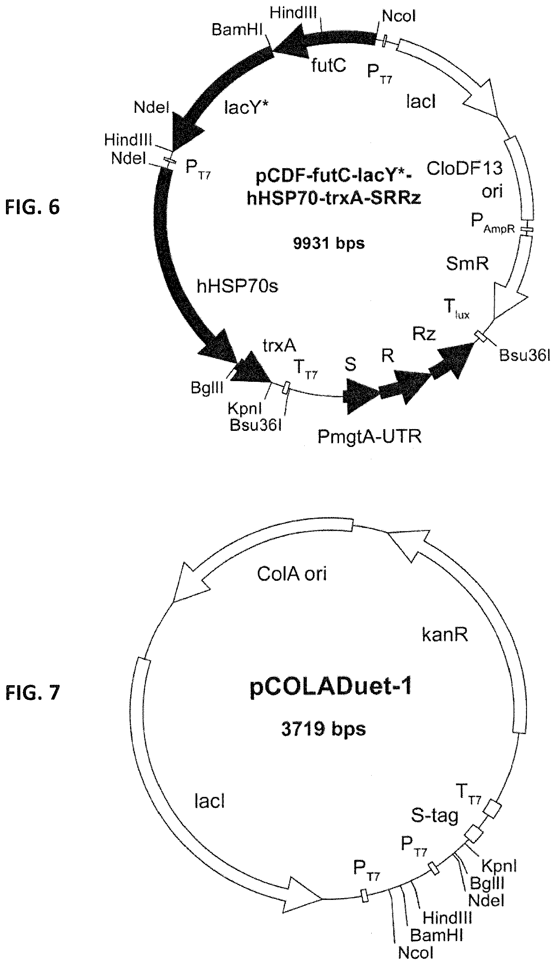

The human *HSP70* gene with codons optimized for *E. coli* is synthesized and provided in a pUC57 vector (GeneScript). The *hHSP70* gene is isolated from this vector via NdeI/BgIII restriction and inserted into the likewise cut vector pCDFDuet-1 (Fig.4), yielding pCDF-hHSP70. The *lacY*-gene is amplified from the genomic DNA of *E. coli* K12-MG1655 using the primers listed in Table 4. For subcloning, the PCR product is inserted into vector pUC19 (Fig.5) via BamHI/HindIII restriction. Site directed mutagenesis is performed to introduce a mutation leading to the amino acid substitution S209I (*IacY**). The *lacY** gene is subsequently digested from vector pUC19-lacY* via BamHI/HindIII and inserted into the likewise cut vector pCDF-hHSP70 to generate pCDF-lacY*-hHSP70. The *futC* gene from *H. pylori,* attached with/without an N-terminal triple Asp-tag and optimized with codons for *E*. *coli,* is provided in a pUC57 vector (GeneScript). The *futC* fragment is isolated from pUC57 via NcoI/BamHI restriction and inserted into pCDF-lacY*-hHSP70/NcoI/BamHI, yielding pCDF-futC-lacY*-hHSP70. The *trxA* gene is amplified from E. *coli* K12-MG1655 and inserted into pCDF-futC-lacY*-hHSP70 via BgIII/KpnI digestion to generate plasmid pCDF-futC-lacY*-hHSP70-trxA. Finally, the SRRz genes from bacteriophage lambda is amplified from *E. coli* BL21 (DE3), introducing a bidirectional *Lux*ICDABEG-terminator from *Vibrio fischeri* at the 3'-end, digested with SacI/PstI and inserted into the similar cut vector pUC19. The promoter of *mgtA* is amplified with the 5'-UTR region from *E*. *coli* BL21 (DE3), digested with Sacl and inserted into pUC19-SRRz-luxTerm/SacI to generate pUC-PmgtA-UTR-SRRz-luxTerm. The gene cassette PmgtA-UTR-SRRz- luxTerm is finally amplified and, after digestion with Bsu36i, inserted into pCDF-futC-lacY*-hHSP70-trxA/Bsu36i to generate pCDF-futC-lacY*-hHSP70-trxA-SRRz (Fig.6). Selected clones is grown in LB with streptomycin (50 µg ml⁻¹) and verified by restriction mapping and DNA sequencing.

### pCOLA-pntAB-rcsA-zwf-gsk

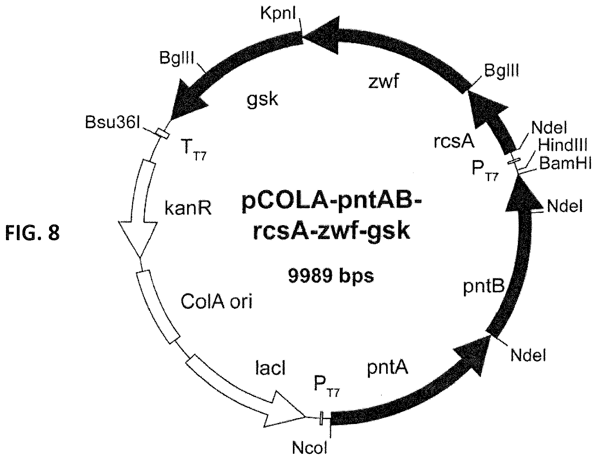

The *rcsA* gene is amplified from *E. coli* K12-MG1655, digested with NdeI/BglII and inserted into the likewise cut vector pCOLADuet-1 (Fig.7) to generate pCOL-rcsA. Next, the *zwf* gene is amplified from *E*. *coli* K12-MG1655 and inserted into pCOLA-rcsA/BglII/KpnI, yielding pCOLA-rcsA-zwf. Subsequently, the *gsk* gene is amplified from *E*. *coli* K12-ATCC10798, digested with KpnI/XhoI and inserted into pCOLA-rcsA-zwf/KpnI/XhoI to generate pCOLA-rcsA-zwf-gsk. The pntAB genes are finally amplified from *E*. *coli* K12-MG1655, digested with NcoI/BamHI and inserted into pCOLA-rcsA-zwf-gsk/NcoI/BamHI to generate vector pCOLA-pntAB-rcsA-zwf-gsk (Fig.8). Selected clones are grown in LB with kanamycin (50 µg ml⁻¹) and verified by restriction mapping and DNA sequencing.

### INDUSTRIAL APPLICABILITY

The present invention provides means for improved production of human milk oligosaccharide (HMO). HMO produced by the microorganisms and production method of the present invention can be widely industrially applied, e.g. as functional ingredients in infant nutrition, medical nutrition, functional foods and animal feed.

## Claims

1. A genetically modified microorganism for the production of human milk oligosaccharide, wherein the microorganism is optionally a bacterium, and preferably *Escherichia coli* (*E. coli*).

2. The genetically modified microorganism according to claim 1, wherein the microorganism comprises an inducible lysis system, wherein the inducible lysis system is optionally auto-inducible.

3. The genetically modified microorganism according to claim 2, wherein the inducible lysis system is Mg²⁺-regulated, and/or wherein the inducible lysis system is optionally regulated by the exogenous free Mg²⁺ concentration, and/or wherein the microorganism optionally comprises a Mg²⁺-regulated promoter, and/or wherein the microorganism further optionally comprises an additional Mg²⁺-regulated element, preferably the 5'UTR of the *mgtA* gene of *Escherichia coli.*

4. The genetically modified microorganism according to any one of claims 2 to 3, wherein the microorganism comprises a lysis genes, wherein the lysis gene is optionally a lysis gene from a bacteriophage.

5. The genetically modified microorganism according to any one of claims 2 to 4, wherein the microorganism comprises a Mg²⁺-regulated promoter and lysis genes, wherein expression of the lysis gene(s) is controlled by the Mg²⁺-regulated promoter, and optionally by an additional Mg²⁺-regulated element.

6. The genetically modified microorganism according any one of claims 1 to 7, wherein the microorganism is unable to cleave lactose into glucose and galactose, and/or wherein optionally the *lac* operon is inactivated, preferably by deletion.

7. The genetically modified microorganism according to any one of claims 1 to 6, wherein the microorganism comprises an exogenous gene encoding a lactose permease, wherein the lactose permease is preferably the *E*. *coli* LacY protein, wherein the lactose permease optionally has A198V and/or S209l mutations.

8. The genetically modified microorganism according to any one of claims 1 to 7, wherein the human milk oligosaccharide is 2'-Fucosyllactose and/or 3'-Fucosyllactose.

9. The genetically modified microorganism according to any one of claims 1 to 8, wherein the microorganism comprises an exogenous gene encoding a fucosyltransferase, wherein the fucosyltransferase is preferably an α-1,2-fucosyltransferase and/or an α-1,3-fucosyltransferase, wherein the gene encoding a fucosyltransferase is optionally a heterologous gene and/or wherein the gene encoding a fucosyltransferase is optionally driven by a heterologous promoter, wherein optionally the gene encoding a fucosyltransferase is codon-optimized for expression in the microorganism.

10. The genetically modified microorganism according to any one of claims 1 to 9, wherein the microorganism comprises a heterologous gene encoding a chaperone, wherein the chaperone is preferably human Hsp70.

11. The genetically modified microorganism according to any one of claims 1 to 8, wherein optionally the *rcsA* gene, preferably derived from *E*. *coli*, is overexpressed; and/or optionally a gene encoding a Lon protease family protein, preferably *E*. *coli* Lon protease, is inactivated, preferably by deletion; and/or optionally the *wcaJ* gene is inactivated, preferably by deletion; and/or optionally the *zwf* gene and the two *pntAB* genes, preferably derived from *E*. *coli*, are overexpressed; and /or optionally the *gsk* gene, preferably derived from *E*. *coli*, is overexpressed; and/or optionally the *trxA* gene, preferably derived from *E*. *coli*, is overexpressed; and/or optionally the *trxB* gene is inactivated, preferably by deletion.

12. A method for producing human milk oligosaccharide, which comprises:
(a) culturing the genetically modified microorganism according to any one of claims 1 to 11 in a medium, wherein the medium further optionally comprises lactose and/or free Mg²⁺, wherein optionally the concentration of the human milk oligosaccharide in the medium at the end of the culture is at least 30 g/L, preferably at least 50 g/L, wherein preferably the human milk oligosaccharide is 2'-Fucosyllactose and/or 3'-Fucosyllactose.

13. The method for producing human milk oligosaccharide according to claim 12, which further comprises:
(b) purifying the human milk oligosaccharide from the culture medium and/or from the microorganism itself, wherein optionally the human milk oligosaccharide is purified from the culture medium after lysis has been induced via the inducible lysis system, and/or wherein optionally the human milk oligosaccharide is purified from the culture medium when the concentration of free Mg²⁺ in the medium is 10 µM or less and/or when the lysis can be directly visualized.

14. Medium obtainable by the method according to claim 12 or human milk oligosaccharide obtainable by the method according to claim 13.

15. Use of the medium or of the human milk oligosaccharide according to claim 14 for the preparation of animal feed.
